# FASCICULE DE BREVET EUROPEEN

(11) **EP 1 514 116 B1**
(45) Date de publication et mention de la délivrance du brevet: **12.08.2009**
(21) Numéro de dépôt: 03760054.1
(22) Date de dépôt: 17.06.2003
(51) Int. Cl.: G01N 33/574, A61K 31/00, A61P 35/00, C12Q 1/68

(54) **PROCÉDÉ DE ANTI-RESISTANCE A L'OXALIPLATINE**
OXALIPLATIN-ANTIRESISTENZVERFAHREN
OXALIPLATIN ANTI-RESISTANCE METHOD

(30) Priorité: 17.06.2002 FR 0207417
(43) Date de publication de la demande: 16.03.2005
(73) Titulaire: CENTRE NATIONAL DE LA RECHERCHE SCIENTIFIQUE (CNRS), 75016 Paris (FR); Université de Montpellier I, 34000 Montpellier (FR); INSTITUT PASTEUR, 75015 Paris (FR)
(72) Inventeur: PAU, Bernard, F-94800 Villejuif (FR); GOURDIER, Isabelle, Centre Nat. Rec. Scientifique, F-75016 Paris (FR); DEL RIO, Maguy, F-34080 Montpellier (FR); CRABBE, Laure, Centre Nat. Rec. Scientifique, F-75016 Paris (FR)
(74) Mandataire: Warcoin, Jacques
(86) Numéro de dépôt international: PCT/FR2003/001842
(87) Numéro de publication internationale: WO 2003/107006

(56) Documents cités:
- MACPHERSON JANET ET AL: "Prior antisense mediated Bcl-xl downregulation in colon cancer cells switches the chemotherapy response from growth arrest to apoptosis, and enhances oxaliplatin cytotoxicity." PROCEEDINGS OF THE AMERICAN ASSOCIATION FOR CANCER RESEARCH ANNUAL, vol. 43, mars 2002 (2002-03), pages 407-408, XP008017902 93rd Annual Meeting of the American Association for Cancer Research;San Francisco, California, USA; April 06-10, 2002, March, 2002 ISSN: 0197-016X
- ARANGO DIEGO ET AL: "Gene interaction determining sensitivity to Oxaliplatin, a chemotherapeutic agent for colon cancer patients." PROCEEDINGS OF THE AMERICAN ASSOCIATION FOR CANCER RESEARCH ANNUAL, vol. 43, mars 2002 (2002-03), page 457 XP008017719 93rd Annual Meeting of the American Association for Cancer Research;San Francisco, California, USA; April 06-10, 2002, March, 2002 ISSN: 0197-016X
- GOURDIER ISABELLE ET AL: "Drug specific resistance to oxaliplatin is associated with apoptosis defect in a cellular model of colon carcinoma." FEBS LETTERS, vol. 529, no. 2-3, 2002, pages 232-236, XP002242753 9 October, 2002 ISSN: 0014-5793

## Description

L'invention concerne le diagnostic de la résistance des cancers, notamment colorectaux, au médicament anti-tumoral "oxaliplatine" (dénomination commune internationale de ce produit, dont le nom commercial est l'Eloxatine).

L'invention concerne également un procédé de selection d' agents « anti-résistance » permettant d'améliorer l'efficacité du traitement à base d'oxaliplatine (en association avec l'oxaliplatine ou en deuxième intention, après développement d'une résistance à l'oxaliplatine).

Les traitements chimiothérapeutiques des cancers colorectaux, malgré la disponibilité de molécules anti-tumorales actives comme l'oxaliplatine, voient leur efficacité très limitée par la survenue fréquente d'une résistance des cellules tumorales aux effets cytotoxiques des médicaments, utilisés seuls ou en combinaison.

La réduction de cette résistance est donc un enjeu majeur pour la santé et l'industrie pharmaceutique. Des traitements à l'oxaliplatine, anticancéreux dont l'administration vise à détruire les cellules cancéreuses, sont notamment décrits dans les documents US 5,716,968 et EP 0 943 331.

L'atteinte de cet objectif, revenant à créer des traitements "anti-résistance" associés aux médicaments anti-tumoraux comme l'oxaliplatine, nécessite l'identification de mécanismes moléculaires jusqu'à présent non élucidés qui gouvernent l'émergence de la résistance à l'intérieur des cellules tumorales.

L'identification de ces mécanismes, inconnus à ce jour dans le cas de la résistance des cancers, notamment des cancers colorectaux, à l'oxaliplatine, vise donc principalement deux applications :
- le diagnostic précoce de la résistance : il s'agit d'éviter des chimiothérapies qui n'auraient aucun bénéfice thérapeutique, alors qu'elles représentent un risque toxique et un coût important,
- le traitement par des médications contrariant ou contournant les mécanismes de résistance.

Il est important de noter qu'il n'existe dans l'art antérieur aucun test précoce de résistance au traitement par l'oxaliplatine.

Résistance à l'oxaliplatine : l'oxaliplatine est un sel de platine possédant un spectre d'activité anti-tumoral beaucoup plus large que les sels de platine conventionnels comme le cisplatine ou le carboplatine. Les mécanismes de résistance au cisplatine ont pu être en grande partie élucidés mais ne rendent pas compte de la résistance à l'oxaliplatine. Plus particulièrement, les dérégulations des systèmes de réparation MMR ou NER associées à la résistance au cisplatine ne confèrent pas de résistance à l'oxaliplatine. La résistance à l'oxaliplatine restait jusqu'à la présente invention inexpliquée. L'oxaliplatine (CgH, 4N204Pt, [(1R, 2R)-1,2-cyclohexanediamine-N, N'] [oxalato (2-)-O, O'] platinum), est un diaminocyclohexane connu pour endommager l'ADN. La présente invention couvre la résistance à l'oxaliplatine, ainsi que le cas échéant à des dérivés de l'oxaliplatine qui donnent lieu également à une résistance.

Deux études, menées sur le même modèle cellulaire de cancer ovarien (lignée ATCC A2780), identifient un mécanisme potentiellement effecteur de la résistance à l'oxaliplatine de ce type de cancer : une augmentation de glutathion intracellulaire, ainsi qu'une diminution d'accumulation intracellulaire de platine et d'adduits ADN-platine sont associés à la résistance à l'oxaliplatine. Mais ces études n'apportent pas de démonstration fonctionnelle à ces identifications. L'hypothèse d'implication du glutathion est soulignée dans le document Cancer Lett. 1996 Jul. 19, 105(1):5-14, Altered glutathione metabolism in oxaliplatin resistant ovarian carcinoma cells (El-akawi Z, Abu-hadid M, Perez R, Glavy J, Zdanowicz J, Creaven PJ, Pendyala L.), Department of Investigational Therapeutics, Roswell Park Cancer Institute, Buffalo, NY 14263, USA.

Une hypothèse de l'intervention de mécanismes de réparation de l'ADN est avancée dans le document Cellular and Molecular Pharmacology of Oxaliplatin, Vol. 1, 227-235, January 2002, Molecular Cancer Therapeutic, (Eric Raymond, Sandrine Faivre, Stephen Chaney, Jan Woynarowski and Esteban Cvitkovic).

On peut toutefois citer le document Arango et al. (Proceedings of the American Association for Cancer Research, vol. 43, page 457, 2002) qui présente une étude sur le mécanisme de la résistance de cellules cancéreuses à l'oxaliplatine et qui décrit certains gènes, comme le gène p53, impliqués dans cette résistance.

On peut également citer le document MacPherson et al. (Proceedings of the American Association for Cancer Research, vol. 43, pages 407-408, 2002) qui décrit l'utilisation *in vitro* d'un agent antisens dirigé contre l'expression de la protéine Bcl-xl pour améliorer l'efficacité de l'oxaliplatine pour le traitement de cellules tumorales.

Toutefois ces études ne permettent pas d'expliquer avec certitude les mécanismes de résistance observée.

Ainsi, l'invention vise à pallier les inconvénients de l'art antérieur, et en particulier à élucider les mécanismes de résistance des cancers, notamment des cancers colorectaux, à l'oxaliplatine, pour pouvoir mettre en oeuvre un diagnostic précoce de la résistance au cours du traitement, et concevoir une démarche pharmacologique rationnelle pouvant aboutir à la mise au point de traitements « anti-résistance » mieux ciblés sur ces mécanismes.

Inversement, la réalisation de tests diagnostiques précoces de la résistance permettra, au moins, d'informer le médecin cancérologue de la nécessité de réorienter le traitement (par exemple en introduisant d'autres médicaments dans le schéma thérapeutique). Le bénéfice en sera la réduction d'effets indésirables et la limitation de dépenses de santé inutiles. De plus, la disponibilité de traitements spécifiques (médicaments, thérapies géniques, ...) contrariant ou contournant la résistance au niveau des mécanismes mis en évidence (apoptose mitochondriale), restaurera l'efficacité de l'oxaliplatine. Le bénéfice sera évidemment médical mais également économique : le gain d'efficacité justifiera le maintien et l'extension de l'utilisation de l'oxaliplatine.

Les inventeurs ont eu à résoudre plusieurs problèmes techniques dont la mise en place d'un modèle expérimental fiable (sélection et caractérisation de lignées cellulaires résistantes à l'oxaliplatine à partir de lignées référencées) et l'exploration de ce modèle (identification de l'altération de l'apôptôse mitochondriale en tant que marqueur de la résistance spécifique à l'oxaliplatine).

Les inventeurs ont réussi à démontrer que la résistance à l'oxaliplatine est associée à l'expression anormale de gènes d'apoptose mitochondriale. L'art antérieur décrit des composés inducteurs d'apoptose agissant directement et spécifiquement au niveau mitochondrial. Toutefois le lien entre l'apoptose mitochondriale (AM) et des mécanismes de résistance à l'oxaliplatine n'est nullement décrit ou suggéré dans l'art antérieur.

Les inventeurs ont ainsi mis au point une méthode de diagnostic de la résistance à l'oxaliplatine, reposant sur la mise en évidence de marqueurs de l'altération de l'apoptose mitochondriale dans les cellules tumorales, par tout moyen approprié : biochimique comme l'immunodétection, génétique comme le séquençage ou la quantification des transcrits.

Ainsi, selon un premier aspect, l'invention concerne un procédé de détection, *in vitro* ou *in vivo,* de la résistance de cellules cancéreuses à un traitement à l'oxaliplatine, comprenant la mesure de l'apoptose mitochondriale de cellules cancéreuses traitées ou pouvant ou devant être traitées à l'oxaliplatine, cette mesure comprenant au moins la mesure de l'expression du gêne d'apoptose mitochondriale codant pour la protéine Bar. Par résistance de cellules cancéreuses traitées à l'oxaliplatine on entend que les cellules cancéreuses, d'un patient où en culture, résistent au traitement à l'oxaliplatine de manière telle que ce traitement n'est pas totalement satisfaisant car ne permettant pas de les détruire à un niveau suffisant.

Ce procédé de détection concerne notamment les cancers colorectaux. Cependant d'autres cancers dont le traitement comprend l'administration d'oxaliplatine font également partie de l'invention, en particulier certains cancers des ovaires, des cellules germinales, du poumon, des voies digestives, de la prostate, du pancréas, de l'intestin grêle, de l'estomac.

Il est décrit ici également un procédé de détection comprenant la mesure de l'expression d'au moins un gène d'apoptose mitochondriale. Par « expression d'au moins un gène d'apoptose mitochondriale », on entend le niveau d'expression d'au moins un gène effecteur ou marqueur d'apoptose mitochondriale. Par gène effecteur, on entend un gène responsable au moins en partie de l'apoptose mitochondriale, cette expression pouvant se traduire notamment par la quantité d'ARNm produite, la quantité de protéines codées par ces gènes, le niveau d'activité de ces protéines. Par exemple un niveau d'apoptose faible peut être dû à la synthèse d'une protéine d'apoptose dont la séquence diffère par rapport à celle d'un patient non résistant, la quantité de protéine étant normale mais son activité biologique étant plus faible. Par gène marqueur on entend un gène qui n'est pas nécessairement impliqué dans les mécanismes de l'apoptose mitochondriale, mais dont le niveau d'expression est corrélé à un niveau d'apoptose déterminé.

Parmi les gènes effecteurs ou marqueurs d'apoptose mitochondriale, on pourra en particulier analyser, en plus des gènes déjà étudiés par les inventeurs (gène Bax notamment), des gènes connus pour leur implication dans les mécanismes d'apoptose mitochondriale, décrits notamment dans le document US 6,268,398 :
- des facteurs initiant ou stimulant la cascade d'apoptose et/ou l'activité de protéases caspases (Thomberry and Lazebnik, Science 281:1312-1316, 1998), tels que le cytochrome c, qui sont libérés suite à un stress oxydatif ;
- des « facteurs inducteurs d'apoptose » décrits dans Murphy, Drug Dev. Res. 46:18-25, 1999 ;
- des facteurs induisant la condensation de la chromatine (Marchetti et al., Cancer Res. 56:2033-38, 1996) qui précède l'apoptose ;
- des protéines Bcl-2, connues pour leur activité anti-apoptose, situées dans la membrane externe des mitochondries (Monaghan et al., J. Histochem. Cytochem. 40:1819-25, 1992), qui protègent les membranes contre le stress oxydatif (Korsmeyer et al., Biochim. Biophys. Act. 1271:63, 1995 ; Nguyen et al., J. Biol. Chem. 269:16521-24, 1994) notamment en bloquant la libération de cytochrome c et l'activation de la caspase 3 (Yang et al., Science 275:1129-1132, 1997 ; Kluck et al., Science 275:1132-1136, 1997).

L'homme du métier dispose de nombreuses techniques appropriées de mesure de l'expression de gènes. On citera par exemple :
- la mesure d'ARNm et d'ADNc, à l'aide de techniques de RT-PCR, de Northern blot, d'hybridation à des banques de cDNA (Sambrook et al., Molecular Cloning-A Laboratory Manual, Cold Spring Harbor Press, New York (1989), de techniques de differential display (Liang et al., 1995, Curr. Op. Immunol. 7:274-280 ; EP 534 858), de techniques utilisant des puces à ADNc ou des oligonucléotides (Eisen, M. B. and P. O. Brown, Methods Enzymol, 303:179-205 (1999) ; Brown, P. O. and D. Botstein, Nat Genet, 21(1 Suppl):33-7 (1999) ; Cheung, V. G., et al., Nat Genet, 21(1 Suppl):15-9(1999)) ;
- la mesure de protéines à l'aide d'analyses western-blot, immunohistochimiques.

Par exemple, la quantification de cytochrome c peut utiliser une méthode spectrophotométrique, immunochimique. La libération de cytochrome c des mitochondries peut être suivie par exemple à l'aide de méthodes immunologiques, de la spectrométrie MALDI-TOF couplée à une capture par affinité (notamment pour l'apocytochrome c et l'holo cytochrome c), du système SELDI (Ciphergen, Palo Alto, USA).

La mesure de l'activité de caspases peut utiliser des tests sur des substrats de caspases (Ellerby et al., 1997 J Neurosci. 17:6165), tels que le peptide marqué synthétique Z-Tyr-Val-Ala-Asp-AFC, Z étant un groupement benzoyle carbonyle et AFC le 7-amino-4-trifluorométhylcoumarin, sur des protéines nucléaires telles que le UI-70 kDa et le DNA-PKcs (Rosen and Casciola-Rosen,1997, J. Cell. Biochem. 64:50 ; Cohen, 1997, Biochem. J. 326:1).

Dans la mesure où le niveau anormalement faible d'apoptose mitochondriale peut être dû à plusieurs gènes, la détection peut impliquer la mesure du niveau d'expression de plusieurs gènes d'apoptose : on peut ainsi déterminer le profil d'expression de plusieurs gènes comparé entre des patients dont on diagnostique la résistance et des patients non résistants. En déterminant des profils d'expression suffisamment précis, le clinicien pourra détecter un phénotype résistant, mais aussi prévoir des résistances pour optimiser la thérapie.

Les gènes d'apoptose mitochondriale peuvent faire partie de l'ADN mitochondrial ou de l'ADN nucléaire.

Selon une réalisation, le procédé de détection comprend la mesure de la quantité et/ou de l'activité de la protéine Bax dans les cellules cancéreuses, ou la mesure des ARNm transcrits codant la protéine Bax.

Selon une réalisation le procédé de détection comprend :
a) la détermination du niveau d'expression d'au moins du gène Bar d'apoptose mitochondriale sur des cellules cancéreuses prélevées chez un patient traitées à l'oxaliplatine;
b) la comparaison du niveau mesure avec un échantillon contrôle de cellules non résistant à l'oxaliplatine.
   Un niveau d'apoptose mitochondriale inférieur indique une résistance. Un niveau d'expression inférieur indique une résistance dans le cas d'un gène effecteur stimulateur de l'apoptose mitochondriale, un niveau d'expression supérieur indique une résistance dans le cas d'un gène effecteur inhibiteur de l'apoptose mitochondriale.
   Les écarts de niveaux d'expression analysés sur un nombre suffisant de patients permettent de déterminer le risque et le degré de résistance, les écarts quantitatifs significatifs observés pouvant être faibles ou élevés selon les gènes impliqués.
   On peut utiliser des échantillons par exemple de biopsies sur un individu atteint d'un cancer à différents moments. Par exemple, un premier échantillon correspond à l'instant du diagnostic et un second échantillon est obtenu à un second instant après un traitement du patient avec une composition comprenant un agent anti-résistance. Le diagnostic peut aussi être effectué suite à une thérapie génique, par exemple pour évaluer le niveau d'apoptose mitochondriale suite au transfert de séquences d'acides nucléiques codant pour des protéines d'apoptose mitochondriale.
   L'invention concerne également un procédé pour la détection de cellules cancéreuses résistantes à l'oxaliplatine comprenant la mise en contact de l'échantillon biologique examiné avec un anticorps capable de reconnaître la protéine Bar d'apoptose ou un fragment biologiquement actif de cette protéine, et la mise en évidence du complexe antigène-anticorps éventuellement formé.
   Pour la mise en oeuvre de tels procédés, on pourra utiliser un kit comprenant :
   a) un anticorps par exemple monoclonal ou polyclonal, ledit anticorps étant capable de reconnaître la protéine d'apoptose ou un fragment biologiquement actif de cette protéine ;
   b) éventuellement les réactifs pour la constitution du milieu propice à la réaction immunologique ;
   c) éventuellement les réactifs permettant la mise en évidence des complexes antigènes-anticorps produits par la réaction immunologique.

   Ainsi, des anticorps peuvent être utilisés pour détecter les protéines d'apoptose sous exprimées. On peut citer, pour une protéine d'apoptose donnée, les anticorps reconnaissant les épitopes de la protéine qui ne sont pas présents dans d'autres protéines.
   Les anticorps destinés à reconnaître spécifiquement un ou plusieurs épitopes des protéines d'apoptose, en particulier la protéine Bax, peuvent être notamment des anticorps monoclonaux, polyclonaux, humanisés, chimériques, des anticorps simples chaînes, des fragments Fab, des fragments Fab'2, des fragments produits par une banque d'expression Fab, des anticorps anti-idiotypiques.
   Les anticorps monoclonaux, population homogène d'anticorps pour un antigène particulier, peuvent être obtenus à l'aide de techniques connues de l'homme du métier telles que la technique des hybridomes de Kohler et Milstein (Nature 256:495-497, 1975; and U.S. Pat. No. 4,376,110), la technique des hybridomes de cellules B humaines (Kosbor et al., Immunology Today 4:72, 1983; Cole et al., Proc. Natl. Acad. Sci. USA 80:2026-2030, 1983), la technique des hybridomes EBV (Cole et al., "Monoclonal Antibodies And Cancer Therapy," Alan R. Liss, Inc. pp. 77-96, 1985). On peut également préparer des anticorps monoclonaux à l'aide de kits de banques de phage display commercialisés par Pharmacia ou Stratagène. Des anticorps chimériques peuvent être obtenus selon une technique de Morrison et al., Proc. Natl. Acad. Sci., USA 81:6851-6855. Des banques d'expression Fab peuvent être construites selon la technique de Huse et al., Science 246:1275-1281, 1989. Des anticorps anti-idiotypiques peuvent être obtenus par la technique de Greenspan et Bona, FASEB J. 7:437-444, 1993.

Selon un autre aspect, l'invention concerne un procédé de détection de la résistance de cellules cancéreuses à un traitement à l'oxaliplatine comprenant détection *in vitro* d'au moins une mutation indicatrice d'une apoptose mitochondriale déficiente dans une région du gène Bax contenant une série de 8 déoxyguanines.

L'identification de telles mutations permet un diagnostic précoce qui permet de mieux cibler la thérapie et d'éviter des traitements inappropriés. Le séquençage comparé de gènes d'apoptose entre des patients dont on diagnostique précocement la résistance et des patients résistants peut également être utilisé.

L'invention concerne également un procédé pour la détection de cellules cancéreuses résistantes à l'oxaliplatine mettant en oeuvre au moins une séquence amorce
ou sonde spécifique du gène Bax d'apoptose mitochondriale, obtenue par des techniques appropriées de construction utilisant des séquences repérées par exemple dans GenBank.

L'intention concerne ainsi un procédé comprenant :
a) éventuellement, l'isolement de l'ADN mitochondrial à partir de l'échantillon biologique à examiner, ou l'obtention d'un ADNc à partir de l'ARN de l'échantillon biologique ou d'ADN génomique ;
b) l'amplification spécifique de l'ADN du a) à l'aide d'au moins une amorce d'amplification du géne Bax d'apoptose mitochondriale.

On pourra utiliser ainsi un kit de diagnostic de la résistance à l'oxaliplatine comprenant des moyens d'extraction de l'ADN mitochondrial de cellules cancéreuses, des moyens de détection et d'amplification d'ARNm de gènes d'apoptose mitochondriale, par exemple du gène Bax, ou d'ADN génomique.

L'invention concerne également un procédé comprenant:
a) la mise en contact d'une sonde nucléotidique du gène Bax d'apoptose mitochondriale avec l'échantillon biologique analysé, l'acide nucléique de l'échantillon ayant le cas échéant préalablement été rendu accessible à l'hybridation, dans des conditions permettant l'hybridation de la sonde et de l'acide nucléique de l'échantillon,
b) la mise en évidence de l'hybride éventuellement formé.

On pourra utiliser un kit de diagnostic de la résistances à l'oxaliplatine comprenant :
a) au moins un compartiment adapté pour contenir et, le cas échéant, contenant une amorce ou une sonde d'un gène d'apoptose mitochondriale tel que le gène Bax ;
b) éventuellement les réactifs nécessaires à la mise en oeuvre d'une réaction d'hybridation ;
c) éventuellement au moins une amorce et les réactifs nécessaires à une réaction d'amplification de l'ADN. Il est également décrit ici un procédé visant à déterminer si un traitement à l'oxaliplatine doit être poursuivi et/ou complété, caractérisé en ce qu'il comprend :

a) l'obtention d'au moins deux échantillons comprenant des cellules cancéreuses issues du patient en cours de traitement à l'oxaliplatine ;
b) la mesure du niveau d'apoptose mitochondriale, par exemple à l'aide de la mesure d'expression de la protéine Bax, dans les échantillons ;
c) la poursuite du traitement lorsque le niveau d'apoptose ne diminue pas lors du traitement

Selon un autre aspect, l'invention concerne un procédé de sélection de composés inhibiteurs de la résistance à l'oxaliplatine, désignés composés anti-résistance, le procédé comprenant l'addition d'au moins un composé candidat à des cellules cancéreuses prélevées sur un patient résistantes à l'oxaliplatine, la comparaison du niveau l'expression du gène Bax d'apoptose mitochondriale en présence et en absence du composé, la déduction de l'effet anti-résistance lorsque le niveau d'expression du gène Bax est est supérieur après l'addition du composé. L'effet anti-résistance est également déduit si le niveau d'expression après addition du composé est supérieur lorsque le gène est un gène stimulateur d'apoptose, et inférieur lorsque le gène est un gène inhibiteur de l'apoptose mitochondriale.

*In vivo* le procédé de sélection peut comprendre, chez un patient traité à l'oxaliplatine et résistant à l'oxaliplatine :
a) l'obtention à un premier instant d'un premier échantillon comprenant des cellules cancéreuses du patient ;
b) l'administration du composé candidat au patient;
c) l'obtention à un second instant d'un second échantillon comprenant des cellules cancéreuses du même patient ;
d) la détermination du niveau d'apoptose mitochondriale et/ou du niveau d'expression d'au moins un gène d'apoptose mitochondriale tel que le gène Bax dans le premier et le second échantillon ;
e) la déduction de l'effet anti-résistance à l'oxaliplatine du composé lorsque le niveau d'apoptose est supérieur dans le second échantillon.

L'effet anti-résistance est également déduit si le niveau d'expression est supérieur dans le second échantillon lorsque le gène est un gène stimulateur d'apoptose, et inférieur si le gène est un gène inhibiteur de l'apoptose mitochondriale. De tels procédés *in vivo* concernent de préférence des composés dérivés de composés déjà identifiés comme anti-résistants.

Par agent anti-résistance on entend un composé capable de diminuer, de préférence de compenser totalement, la résistance des patients à l'oxaliplatine. Ces agents anti-résistance sont destinés à rétablir le niveau normal d'expression d'au moins un gène d'apoptose mitochondriale, soit directement, soit indirectement par exemple par l'activation ou l'inhibition de molécules régulatrices de l'expression de ces gènes. Un agent anti-résistance pourra par exemple bloquer l'activité d'un composé responsable d'une inhibition anormale de l'activité de gènes d'apoptose au niveau de la transcription, de la traduction, ou de l'activité d'une protéine.

On peut rechercher des composés candidats notamment parmi des petites molécules, des polypeptides (par exemple oligopeptides, anticorps, fragments d'anticorps), des acides nucléiques. Les procédés de criblage d'agents anti-résistance à l'oxaliplatine font typiquement intervenir des banques de molécules connues de l'homme du métier telles que des banques de substances biologiques (protéines notamment), des banques de substances synthétiques.

Des banques de composés peuvent se présenter sous forme de solution (e.g., Houghten, 1992, Biotechniques 13:412-421), sur des billes (Lam, 1991, Nature 354:82-84), sur des puces (Fodor, 1993, Nature 364:555-556). On peut également utiliser des banques décrites dans les documents US 5,292,646 et 5,270,281.

On pourra étudier en particulier l'effet de composés déjà connus de l'homme du métier comme stimulateurs de l'apoptose mitochondriale, tels que le TNF (facteur de nécrose des tumeurs), le FasL, le glutamate, l'Herbimycin A (Mancini et al., J. Cell. Biol. 138:449-469, 1997), le Paraquat (Costantini et al., Toxicology 99:1-2, 1995), des inhibiteurs de protéines kinase tels que la Staurosporine, la Calphostin C, les dérivés de la d-érythro-sphingosine, le chlorure de Chélérythrine, des inducteurs de MAP kinase tels que l'Anisomycine, des inducteurs de MPT catégorie dont fait partie la protéine Bax (Jurgenmeier et al., Proc. Natl. Acad. Sci. U.S.A. 95:4997-5002, 1998).

Parmi les tests permettant de mesurer le niveau d'apoptose mitochondriale, on peut citer la mesure de l'activité enzymatique de complexes mitochondriaux ETC I, II, III, IV et de l'ATP synthétase, la mesure de la consommation mitochondriale d'oxygène (Miller et al., J. Neurochem., 67:1897, 1996), la mesure de l'état d'oxydation du cytochrome c mitochondrial à 540 nm, la mesure du stress oxydatif en présence et en absence de l'agent anti-résistance.

Il est également décrit ici l'utilisation d'au moins un agent anti-résistance stimulateur de l'apoptose mitochondriale pour la préparation d'un médicament chez des patients présentant ou susceptibles de présenter une résistance à l'oxaliplatine. Par patient résistant on entend un patient présentant des cellules cancéreuses résistantes à l'oxaliplatine. On peut utiliser un tel agent anti-résistance chez des patients présentant une réponse partielle au traitement à l'oxaliplatine pour améliorer l'efficacité du traitement.

Selon une réalisation les composés anti-résistance sont issus d'un procédé de sélection tel que décrit précédemment. L'homme du métier dispose de tests suffisamment décrits dans la demande pour sélectionner ces composés ; l'invention couvre donc également l'utilisation de ces composés, même si la structure chimique précise des composés n'est pas totalement identifiée : si un composé testé répond aux critères de sélection (en particulier stimulation de l'apoptose, augmentation de l'expression d'au moins un gène stimulateur d'apoptose, diminution de l'expression d'au moins un gène inhibiteur d'apoptose.), alors l'homme du métier pourra l'utiliser pour la préparation d'un médicament anti-résistance à l'oxaliplatine sans avoir nécessairement besoin de connaître sa structure chimique.

Le traitement ciblera plus spécialement les cellules cancéreuse ayant acquis la résistance à l'oxaliplatine. Le traitement vise à rétablir un niveau d'expression ou d'activité de gènes impliqués dans l'apoptose mitochondriale suffisant pour que les cellules cancéreuse résistantes réengagent plus activement ce processus. On recherchera une apoptose normale similaire à celle de cellules cancéreuses non résistantes, ou au moins une augmentation de l'apoptose mitochondriale suffisante pour réduire les symptômes cliniques.

Le traitement du patient fera intervenir typiquement l'association de l'oxaliplatine et d'au moins un agent anti-résistance, selon une administration qui peut être simultanée, séparée ou étalée dans lé temps. La quantité d'agents anti-résistance à administrer aux patients doit être suffisante pour être thérapeutiquement efficace, pour réduire au moins partiellement la résistance à l'oxaliplatine. Le traitement combinant l'oxaliplatine et au moins un agent de anti-résistance à l'oxaliplatine, chez un patient résistant, vise de manière préférentielle à obtenir une efficacité thérapeutique au moins égale à celle d'un traitement à l'oxaliplatine chez un patient non résistant.

Il est décrit également une méthode de traitement d'un patient résistant à l'oxaliplatine ou susceptible de présenter une résistance à l'oxaliplatine, comprenant l'administration d'au moins un composé stimulateur de l'apoptose mitochondriale.

Il est décrit également un procédé pour inhiber une résistance à l'oxaliplatine chez l'homme, comprenant l'administration d'un composé capable de stimuler sélectivement l'apoptose mitochondriale de cellules cancéreuses, chez un patient requérant un tel traitement anti-résistance.

La toxicité et l'efficacité thérapeutique des agents anti-résistance peuvent être déterminées par des techniques standard d'expérimentation sur les cultures de cellules ou des animaux de laboratoire. La transposition sur des patients humains connaissant ces données est obtenue à l'aide de méthodes appropriées.

Une formulation pourra comprendre de l'oxaliplatine typiquement dans une quantité de 1 à environ 10 mg/ml, de préférence 1 à 5 mg/ml, et encore préférée de 2 à 5 mg/ml. Les doses d'oxaliplatine administrées au patient résistant seront typiquement de l'ordre de 10 mg/m2/jour à 250 mg/m2/jour, de préférence 20 mg/m2/jour à 200 mg/m2/jour, de préférence entre 50 et 150 mg/m2/jour.

L'administration peut être répétée pendant des cycles de 1 à 5 jours espacés d'un intervalle de 1 à 5 semaines. Pour des patients présentant une plus forte résistance, le clinicien déterminera la dose d'oxaliplatine appropriée, la dose d'agents anti-résistance, la durée du traitement.

On pourra associer le cas échéant à l'oxaliplatine et à l'agent anti-résistance au moins un composé connu de l'homme du métier pour renforcer l'efficacité e/ou la stabilité de l'oxaliplatine ; de tels agents sont décrits dans les documents EP 0 943 331 et WO 01/66102.

L'oxaliplatine sera typiquement associée à un transporteur pharmaceutiquement acceptable, tel qu'un solvant approprié. Le transporteur sera en général de l'eau, ou un ou plusieurs solvants, ou un mélange d'eau et d'un ou plusieurs solvants appropriés. On pourra préférer de l'eau pure stérile pour injection et parmi les solvants : les polyalkylène glycols tels que le polyéthylène glycol, le polypropylène glycol, le polybutylène glycol et analogues, l'éthanol, le polymère 1-vinyl-2-pyrrolidone, des solutions de sucres pharmaceutiquement acceptables telles que le lactose, le dextrose, le sucrose, le mannose, le mannitol, les cyclodextrines ou analogues. Le pH des formulations en solution d'oxaliplatine est typiquement de 2 à 5, de préférence de 3 à 4,5.

Les formulations seront administrées aux patients par des voies conventionnelles appropriées, typiquement par voie parentérale (par exemple intraveineuse, intrapéritonéale et analogues). L'administration intraveineuse se fera par exemple sur une période de 12 heures à 5 jours. Le pourcentage du composé actif dans les formulations mixtes selon l'invention comprenant l'oxaliplatine et au moins un agent de résistance, est adapté selon le dosage et le degré de résistance à l'oxaliplatine en particulier. Le dosage approprié pour un patient particulier sera déterminé notamment en fonction du type d'administration choisi, de la durée du traitement, de la taille, de l'âge, de la condition physique du patient, du degré de résistance à l'oxaliplatine, de la réponse du patient à la composition.

L'incorporation de l'agent anti-résistance dans la composition d'oxaliplatine s'effectue par les techniques appropriées.

Pour une administration orale à l'aide de pastilles, poudres, granules et analogues, on pourra utiliser des excipients tels que le lactose, le chlorure de sodium, le sucrose, le glucose, l'urée, l'amidon, le calcium, le kaolin, la cellulose cristalline, l'acide salicyllique, la méthyle cellulose, le glycérol, l'alginate de sodium, la gomme arabique et analogues. On pourra utiliser des agents de liaison habituels tels que des solutions de glucose, des solutions d'amidon, des solutions de gélatine. On pourra utiliser des désintégrants tels que l'amidon, l'alginate de sodium, la poudre d'agar, le carbonate de calcium. Parmi les agents absorbants, on pourra utiliser l'amidon, le lactose, le kaolin, la bentonite. Parmi les lubrifiants, on pourra utiliser le talc purifié, les sels d'acides stéariques, le polyéthylène glycol.

On prendra soin pour la formulation d'éviter des problèmes éventuels dus à l'association d'oxaliplatine et d'un agent anti-résistance tels que les problèmes de précipitation des composés.

Une composition thérapeutique d'oxaliplatine comprendra typiquement 0,005 % à 95 %, de préférence 0,5 à 50 % d'oxaliplatine et d'agents anti-résistance.

Sur le plan dû mécanisme d'action, selon une réalisation, l'agent anti-résistance est un agent stimulateur de l'expression d'au moins un gène d'apoptose mitochondriale.

Selon une réalisation, l'agent anti-résistance est une molécule capable d'inhiber l'expression de gènes inhibiteurs de l'apoptose mitochondriale. On pourra utiliser des oligonucléotides antisens complémentaires d'ARNm codant des molécules inhibitrices de l'expression de gènes effecteurs d'apoptose. L'oligonucléotide antisens se liera spécifiquement à l'ARNm de telles molécules inhibitrices en inhibant leur traduction. La complémentarité devra être suffisante pour que l'hybridation avec l'ARNm de la molécule inhibitrice conduise à la formation d'hybrides stables. Typiquement, on utilisera des antisens d'une longueur comprise entre 6 et 50 nucléotides typiquement d'au moins 10 à 20 nucléotides. Les antisens peuvent être synthétisés par des méthodes connues de l'homme du métier en utilisant des nucléotides modifiés pour augmenter la stabilité du duplex antisens/sens. On peut utiliser par exemple les nucléotides modifiés suivants : 5-fluorouracile, 5-bromouracile, 5-chlorouracile, 5-iodouracile, hypoxanthine, xanthine, 4-acétylcytosine, 5-(carboxyhydroxylméthyl) uracile, 5-carboxyméthyl aminométhyl-2-thiouridine, 5-carboxyméthylaminométhyluracile, dihydrouracile, bêta-D-galactosylquéosine, inosiné, N6-isopentenyladénine, 1-méthylguanine, 1-méthyl inosine, 2,2-diméthylguanine, 2-méthyladénine, 2-méthylguanine, 3-méthylcytosine, 5-méthylcytosine, N6-adénine, 7-methylguanine, 5-méthylaminométhyluracile, 5-méthoxy amininométhyl-2-thiouracile, beta-D-mannosylqueosine, 5'-méthoxycarboxyméthyl uracile, 5-méthoxyuracile, 2-méthylthio-N6-isopentenyladénine, 5-méthyl-2-thiouracile, 3-(3-amino-3-N-2-carboxypropyl) uracile, 2,6-diaminopurine. Les antisens peuvent aussi être produits biologiquement à l'aide d'un vecteur d'expression dans lequel l'antisens a été sous cloné dans une orientation antisens.

L'antisens pourra le cas échéant être conjugué avec des molécules peptidiques facilitant son transport ou son activité au niveau du site d'action ciblée. On peut injecter les molécules antisens directement dans une zone cible du tissu, l'antisens peut être lié à des molécules telles que des peptides ou des anticorps capables de se lier spécifiquement à des récepteurs exprimés à la surface des cellules cibles.

L'administration d'antisens sera telle que ces molécules puissent agir à un niveau suffisant dans les mitochondries.

Il est décrit également une composition pharmaceutique comprenant de l'oxaliplatine et au moins un agent anti-résistance capable de stimuler l'apoptose mitochondriale, en stimulant l'expression de gènes d'apoptose mitochondriale ou en bloquant des effecteurs responsables de la résistance.

Selon une réalisation, l'agent anti-résistance est un agent de régulation-stimulation de l'expression du gène Bax, et/ou un agent de blocage d'effecteurs de résistance.

L'expression de gènes d'apoptose mitochondriale peut être augmentée par le transfert d'acides nucléiques contenant une séquence codante pour le gène d'apoptose et/ou une séquence régulatrice, à l'aide de techniques de transfert appropriées pour les mitochondries. Ces séquences peuvent être liées dans des vecteurs d'expression et transférées dans les cellules, par exemple à l'aide de plasmides. L'acide nucléique inséré dans le vecteur peut coder la séquence complète de la protéine d'apoptose ou un fragment biologiquement actif ayant une activité de préférence d'au moins 50, 70, 90, 95 % de l'activité de la protéine d'apoptose complète.

Les acides nucléiques utilisables dans les vecteurs d'expression peuvent être liés opérationnellement à des séquences régulatrices telles qu'une séquence promoteur ou enhancer qui stimule leur expression. Ces séquences régulatrices peuvent être celles associées naturellement aux gènes codant pour des protéines d'apoptose.

L'homme du métier connaît un grand nombre de techniques appropriées pour le transfert d'acides nucléiques dans les cellules par des vecteurs notamment plasmidiques, telles que la technique liposome-polybrène, la transfection DEAE dextran (Felgner et al., Proc. Natl. Acad., Sci. USA, 84:7413,1987 ; Ono et al., Nuerosci. Lett. 117:259, 1990 ; Brigham et al., Am. J. Med. Sci. 298:278, 1989), l'électropration (Neumann et al., EMBO J., 7:841, 1980), la précipitation au phosphate de calcium (Graham et al., Virology, 52:456, 1973 ; Wigler et al., Cell., 14:725, 1978 ; Felgner et al., supra), la microinjection (Wolff et al., Science, 247:1465, 1990), les techniques biolistiques. On utilisera de préférence des vecteurs appropriés pour un transfert de gènes au niveau mitochondrial, par exemple le virus HBV (virus de l'hépatite B), transfert décrit par exemple dans le document US 6,100,068. Ainsi, le traitement de la résistance à l'oxaliplatine repose sur l'utilisation de nouveaux procédés thérapeutiques, en particulier le recours à des substances chimiques et/ou à des thérapies géniques, capables de réduire la résistance en restaurant l'activation de l'apoptose mitochondriale normalement provoquée par l'oxaliplatine dans les cellules tumorales des cancers colorectaux.

La présente invention décrit une cellule HCT116/S telle que déposée le 16 juin 2003, sous le numéro : I-3051, auprès de la Collection Nationale de Cultures de Microorganismes (CNCM), Institut Pasteur, Paris, France.

La lignée dénommée HCT116/S est issue d'un sous-clonage de la lignée sauvage HCT116 de l'ATCC (afin de s'assurer de la clonalité de ces cellules). Cette lignée HCT116/S a fait l'objet d'un dépôt le 16 juin 2003, sous le numéro : I-3051, auprès de la Collection Nationale de Cultures de Microorganismes (CNCM) de l'Institut Pasteur, Paris, France, dans le cadre du traité de Budapest.

Il est également décrit l'utilisation d'une cellule HCT116/S telle que déposée à la CNCM le 16 juin 2003, sous le numéro : I-3051, ou de toute cellule dérivée de cette cellule HCT116/S, pour étudier la corrélation entre la résistance de cellules cancéreuses, de préférence colorectales, à un traitement anti-cancéreux et l'expression d'un gène d'apoptose mitochondriale.

Par cellule dérivée de cette cellule HCT116/S telle que déposée à la CNCM le 16 juin 2003, sous le numéro : I-3051, on entend désigner ici notamment, toute cellule fille issue de cette lignée HCT116/S, ou toute cellule variante issue de cette lignée HCT116/S ayant de préférence acquise une résistance à un composé, tel qu'un composé anti-cancéreux comme l'oxaliplatine, ou ayant retrouvé une sensibilité à un tel composé (cf. par exemple les lignées cellulaires issues de HCT116/S décrites ci-après dans le paragraphe « Mise en place modèle expérimentale »).

De telles cellules HCT116/S telle que déposée à la CNCM le 16 juin 2003, sous le numéro : I-3051, ou leurs cellules dérivées pourront également être utilisées pour la mise en évidence et l'identification d'un gène d'apoptose mitochondriale dont l'expression est liée à la résistance de cellules cancéreuses, de préférence colorectales, à un traitement anti-cancéreux, notamment à un traitement à l'oxaliplatine. De telles cellules HCT116/S telle que déposée à la CNCM le 16 juin 2003, sous le numéro : I-3051, ou leurs cellules dérivées pourront également être utilisées pour la sélection d'un composé capable de stimuler l'apoptose mitochondriale d'une cellule cancéreuse, ledit composé étant destiné à être associé à un agent anti-cancéreux pour lequel ladite cellule cancéreuse est résistante, de préférence ledit agent anti-cancéreux pour lequel ladite cellule cancéreuse est résistante est l'oxaliplatine et, le cas échéant, ladite cellule est une cellule cancéreuse colorectale.

Un tel procédé comprendra notamment une étape dans laquelle, ledit composé à tester et l'agent anti-cancéreux pour lequel ladite cellule cancéreuse est résistante, seront mis en présence desdites cellules HCT116/S ou leurs cellules dérivées mis, puis l'observation de la résistance de ces cellules, notamment en étudiant l'activité de gènes d'apoptose mitochondriale, telle que l'activité de Bax et/ou Bak, ou encore de gènes impliqués dans l'apoptose mitochondriale tels que cités ci-avant.

D'autres objets et avantages de l'invention apparaîtront à la lecture de la description détaillée qui suit, illustrée par les figures suivantes :
- la figure 1 démontre que la lignée HCT116R, résistante à l'oxaliplatine, n'exprime pas la protéine Bax ;
- les figures 2A à 2D démontrent que les lignées HCT116R et SW620R résistent à l'induction apoptotique telle qu'elle est provoquée par l'oxaliplatine dans les lignées HCT116 et SW620 d'origine ;
- les figures 3A et 3B démontrent que les lignées HCT116R et SW620R résistent également à l'induction apoptotique mitochondriale directe, qui peut être obtenue sous l'effet des agents arsenic et lonidamine ;
- les figures 4A à 4C démontrent que la sensibilité à l'oxaliplatine est associée au degré d'activation de Bax ;
- les figures 5A à 5E démontrent que la sensibilité à l'oxaliplatine est associée au degré d'activation de Bak.

### Mise_en_place du modèle expérimental :

Les travaux ont été réalisés *"in vitro"* sur des lignées cellulaires de cancer colorectal (CCR) obtenues auprès de la collection internationale gérée aux Etats-Unis (ATCC) et re-clonées au laboratoire. Ces lignées sont référencées, en particulier par l'Institut américain de recherche sur le cancer (NCI/NIH), comme standards pour l'évaluation pharmacologique des drogues anti-tumorales.

A partir de ces lignées, sensibles à l'effet cytotoxique de l'oxaliplatine, les inventeurs ont isolé des lignées dérivées capables de résister spécifiquement à l'oxaliplatine (et non aux autres médicaments que sont le cisplatine et l'irinotécan), en exposant ces cellules à des concentrations croissantes d'oxaliplatine, dans un schéma adapté à l'acquisition de la résistance. Les résultats présentés dans la demande concernent les lignées d'origine, HCT116 et SW620, ainsi que leurs dérivées HCT116R (dénommée aussi ci-après HCT116/R) et SW620R (dénommée aussi ci-après SW620/R), respectivement 70 et 20 fois plus résistantes que les lignées d'origine.

Concernant la lignée référencée ATCC HCT116, deux lignées sont ainsi décrites dans l'exemple 1 ci-après :
- la lignée dénommée HCT116, qui est dénommée HCT116/S dans l'exemple 2, est issue d'un sous-clonage de la lignée sauvage Hot116 de l'ATCC (afin de s'assurer de la clonalité de ces cellules). Cette lignée HCT116JS a fait l'objet d'un dépôt le 16 juin 2003, sous le numéro : I-3051, auprès de la Collection Nationale de Cultures de Microorganismes (CNCM) de l'Institut Pasteur, Paris, France, selon les dispositions du traité de Budapest, ceci conformément à la Règle 6.1 ;
- la lignée dénommée HCT116R ou HCT116%R, qui est dénommée HCT116/R2 dans l'exemple 2, dérivée de la lignée HCT116 après acquisition de résistance à l'oxaliplatine et clonage (correspond au clone 70 fois plus résistant que la lignée sensible de référence HCT116/S).

Dans l'exemple 2, trois autres variants cellulaires sont décrits. Il s'agir
- de la lignée HCT116/R1 dérivée de la lignée HCT116 après acquisition de résistance à l'oxaliplatine et clonage (correspondant à un clone 30 fois plus résistant que la lignée sensible de référence HCTI 16/S),
- du variant HCT116/Rev1 dérivé de la lignée HCT116/R1 après culture en absence d'oxaliplatine pendant 6 mois. Ce variant est caractérisé par un retour au niveau initial de sensibilité (sensibilité à l'oxaliplatine comparable à HCT116/S),
- du variant HCT116/Rev2 dérivé de la lignée HCT116/R2 après culture en absence d'oxaliplatine pendant 15 mois. Ce variant est caractérisé par une perte seulement partielle de résistance à l'oxaliplatine (variant HCT116/Rev2 est 16 fois plus résistant que la lignée HCT116/S).

Les lignées HCT116/R1 et Rev1 ne sont pas porteuses de la mutation homozygote du gène Bax identifiée sur HCT116/R2 (contrôlé par séquençage de la région contenant les codons 38 à 41). Le niveau d'expression de Bax est équivalent pour HCT116/S, R1 et Rev1. Le variant HCT116/Rev2 conserve la mutation homozygote identifiée sur HCT116/R2 ainsi que l'absence d'expression de Bax caractéristique de cette mutation.

### EXEMPLE 1 : Résistance à l'oxaliplatine des lignées HCT116R et SW620R dérivées des lignées de CCR HCT116 et SW620, et résultats

**Tableau 1**

| «Les lignées HCT116R et SW620R dérivées des lignées de CCR HCT116 et SW620 sont spécifiquement résistantes à l'oxaliplatine » | | | |
|---|---|---|---|
| | **IC₅₀(µM)^{a}** | | |
| Lignée cellulaire | Oxaliplatine | Cisplatine | Irinotécan |
| HCT116 | 0,32 ±0,08 **(1,0)^{b}** | 4,7±1,8 **(1,0)** | 7,7 ± 3,8 **(1,0)** |
| HCT116/R | 21,9 ± 6,3 **(68,4)** | 13,4 ± 6,6 **(2,9)** | 9,5 ±4,2 **(1,2)** |
| SW620 | 3,4±0,6 **(1,0)** | 7 ±1,7 **(1,0)** | 23,3 ±0,6 **(1,0)** |
| SW620/R | 62,3±12,9 **(18,3)** | 9±1,7 **(1,4)** | 11,7±2,5 **(0,5)** |

| | | | |
|---|---|---|---|
| ^{a} La concentration inhibitrice 50 ou IC est la concentration de médicament qui diminue la croissance cellulaire de 50 %. Les valeurs d'IC₅₀ ont été mesurées par le test colorimétrique wst1 après incubation du médicament pendant 48 heures. Les valeurs correspondent à la moyenne ± SD obtenue à partir d'au moins trois expériences indépendantes. ^{b} Les nombres entre parenthèses correspondent à la résistance relative, déterminée par le rapport de l'IC₅₀ du clone résistance divisée par l'IC₅₀ du clone parental. | | | |

Le tableau 1 montre que les lignées HCT116R et SW620R sont approximativement 70 fois et 20 fois plus résistantes à l'oxaliplatine que les lignées dont elles dérivent. Elles présentent très peu ou pas de résistance croisée au cisplatine et à l'irinotécan. Leur résistance est donc spécifique de l'oxaliplatine.

Le travail a été mené en parallèle sur deux modèles cellulaires de fonds génétiques différents de façon à pouvoir renforcer la signification des résultats observés ; ainsi, la lignée SW620 est issue d'une métastase et possède une protéine régulatrice p53 mutée alors que la lignée HCT116 est issue d'une tumeur primitive à instabilité microsatelllite et possède une protéine p53 sauvage. L'observation de l'altération de l'apoptose mitochondriale associée au phénotype résistant (voir plus loin), dans deux contextes cellulaires différents, permet de généraliser les résultats et donc apportent une forte probabilité de son impact médical.

### Exploration du modèle expérimental

Les démonstrations essentielles ont été apportées sur ces deux lignées distinctes, de façon à corroborer le caractère universel de l'invention. Quelques études complémentaires ont été limitées à la lignée HCT116 et à sa dérivée HCT116R.

Les mécanismes moléculaires de résistance à l'oxaliplatine des cellules de CCR étant inconnus, les inventeurs ont étudié comparativement l'expression génique des phénotypes sensible et résistant dans le modèle HCT116 (analyse de transcriptome). Les inventeurs ont réussi à identifier un abaissement marqué du taux d'ARN messagers de certains gènes lié à l'apoptose, en particulier du gène Bax impliqué dans la voie dite "Apoptose mitochondriale" (AM).

Ces observations ont été renforcées par l'analyse biochimique (l'immuno-empreinte signale la disparition de l'expression de la protéine Bax) et par le séquençage du gène Bax (dans la lignée HCT116R, une mutation homozygote du gène Bax supprime son expression).

### Figure 1 :

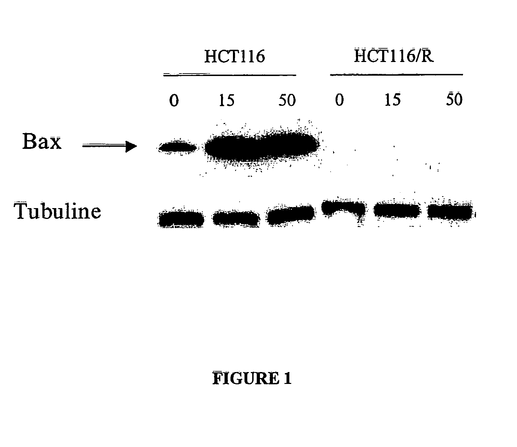

La figure 1 montre que la lignée HCT116R n'exprime pas la protéine Bax, avec ou sans traitement à l'oxaliplatine, alors que la lignée HCT116 d'origine l'exprime sans traitement et la sur-exprime après traitement par l'oxaliplatine. Un séquençage a démontré que la lignée HCT116R est mutante homozygote (délétion d'une déoxyguanosine) dans une région du gène Bax contenant une série de 8 déoxyguanosines (codons 38 à 41), ce qui interdit son expression par décalage du cadre de lecture. La lignée HCT116 d'origine étant hétérozygote G8/G7, elle exprime donc normalement le gène Bax.

Légende de la figure 1 : détection de Bax par Western-blotting en absence, ou sous l'effet d'un traitement, à l'oxaliplatine dans le modèle HCT116. Les cellules sont non traitées ou traitées par l'oxaliplatine à raison de 15 µM pendant 48 h (ou 50 µM pendant 24 h) avant la préparation des lysats cellulaires. L'expression de la tubuline est utilisée comme contrôle de dépôts protéiques équivalents.

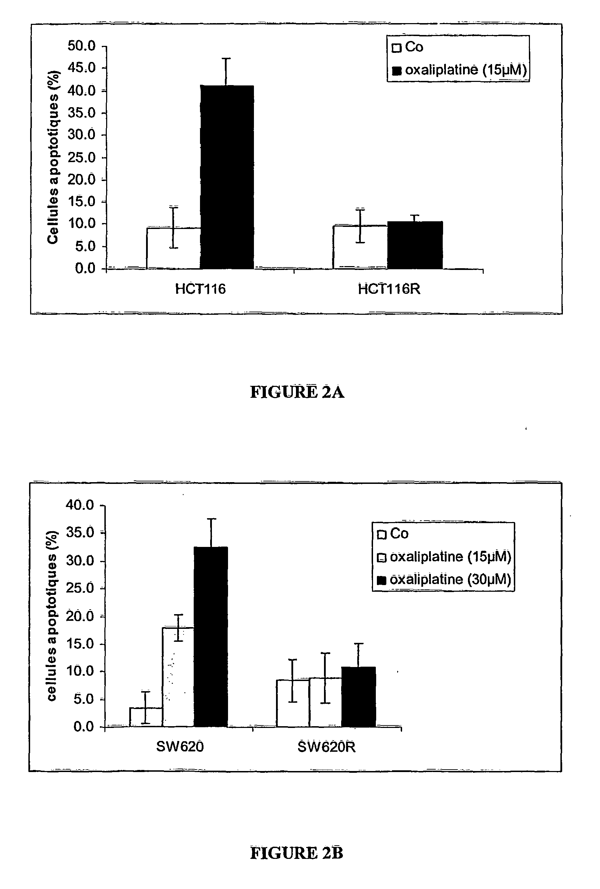

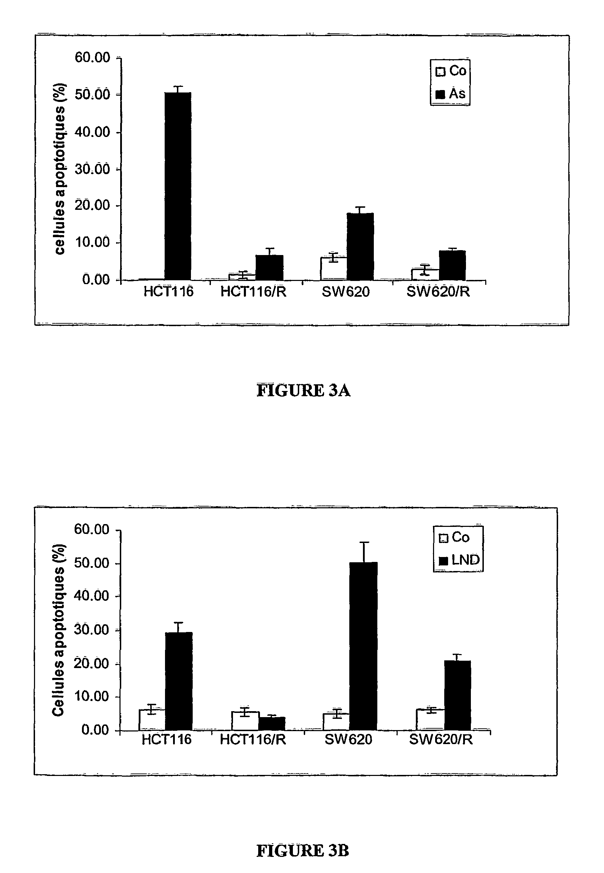

Les inventeurs ont focalisé les travaux sur l'étude fonctionnelle de cette voie, en liaison avec la résistance à l'oxaliplatine. Les principaux résultats obtenus sont les suivants :
- Dans un premier temps, les inventeurs ont démontré que les lignées HCT116R et SW620R, comparativement aux lignées d'origine, sont résistantes à l'induction d'apoptose par l'oxaliplatine. Les inventeurs ont également vérifié sur le modèle HCT116 que cette résistance à l'apoptose est spécifiquement développée vis à vis de l'oxaliplatine, puisque la lignée HCT116R reste sensible à l'induction d'apoptose par un autre médicament anti-CCR (l'irinotécan) dont le mécanisme d'action est différent. Figures 2A à 2D :

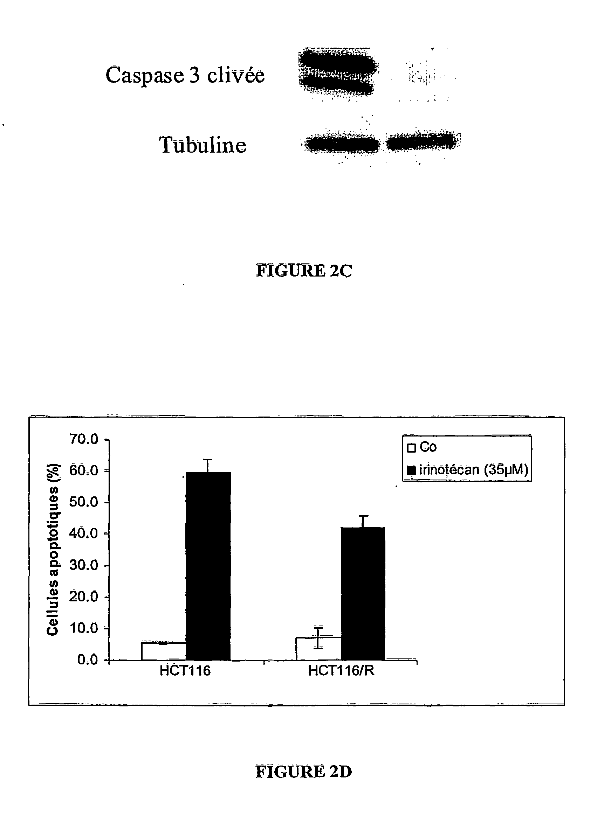

   Les figures 2A à 2D montrent que les lignées HCT116R et SW620R résistent à l'induction d'apoptose par l'oxaliplatine. Cette résistance a été spécifiquement développée vis-à-vis de l'oxaliplatine : la lignée HCT116R ne résiste pas à l'induction apoptotique provoquée par un médicament anti-CCR au mode d'action différent, l'irinotécan (cf. figures 2A, 2B, 2D). La résistance à l'induction d'apoptose par l'oxaliplatine, observée par cytofluorimétrie après marquage par l'annexine V, est confirmée par le défaut d'activation de la caspase 3 (figure 2C). Légende des figures 2A à 2D : les cellules HCT116 (et R) et SW620 (et R) sont traitées, durant 48 heures préalablement à la détermination du degré d'apoptose, par l'oxaliplatine (figures 2A et 2B) ou un autre médicament anti-CCR, l'irinotécan pour les cellules HCT116 et HCT116R (figure 2D). Un contrôle est réalisé sans contact avec aucun médicament (Co). Le degré d'apoptose est alors déterminé en cytofluorométrie utilisant le marquage par l'annexin V. Indépendamment, l'activation de la protéine effectrice d'apoptose Caspase 3 a été évaluée dans les cellules HCT116 et HCT116R après traitement durant 24 heures avec l'oxaliplatine afin de valider l'entrée en apoptose des cellules telle qu'observée par cytofluorométrie (figure 2C).
- Dans un deuxième temps, les inventeurs ont démontré que la résistance à l'apoptose induite par l'oxaliplatine des lignées HCT116R et SW620R s'accompagne d'une résistance à l'induction de l'apoptose par deux agents chimiques connus pour être des activateurs directs de l'AM (trioxide d'arsenic et lonidamine). Figures 3A et 3B :
   Les figures 3A et 3B montrent que les lignées HCT116R et SW620R sont résistantes à l'induction apoptotique sous l'effet des activateurs directs de l'AM que sont l'arsenic et la lonidamine.

   Légende des figures 3A et 3B : les cellules HCT116 (et R) et SW620 (et R) sont traitées, préalablement à le détermination du degré d'apoptose, durant 24 heures par le trioxide d'arsenic (As), la lonidamine (LND) ou sont laissées sans traitement (contrôle, Co). Le degré d'apoptose est alors déterminé en cytofluorométrie après marquage au colorant "Mitocapture" qui fluoresce différemment dans les cellules apoptotiques et les cellules intactes (en relation avec l'intégrité mitochondriale).

Les inventeurs ont ainsi démontré que des altérations génétiques, biochimiques et fonctionnelles de l'apoptose sont associées à la résistance à l'oxaliplatine. Elles concernent deux lignées cellulaires de CCR sélectionnées séparément pour leur résistance spécifique à l'oxaliplatine. La pertinence de cette sélection est validée par les caractéristiques suivantes :
- L'acquisition de résistance à l'oxaliplatine est spécifique puisqu'elle ne s'accompagne pas d'une acquisition de résistance au cisplatine (molécule apparentée et présentant très fréquemment une résistance croisée à l'oxaliplatine) ou à l'irinotécan (autre molécule indiquée dans le traitement du CCR en alternative de l'oxaliplatine ou en association).
- La résistance à l'oxaliplatine, ainsi que les altérations fonctionnelles (résistance à l'apoptose) sont observées pour une concentration d'oxaliplatine équivalente au pic plasmatique chez l'homme au cours des traitements.

Les inventeurs ont démontré que la résistance à l'apoptose s'exerce au niveau mitochondrial. Cela est notamment démontré par des essais avec des inducteurs directs de l'AM. Ces altérations sont donc des marqueurs diagnostics de la résistance des cancers colorectaux à l'oxaliplatine. De plus, il est probable que la modulation pharmacologique de la voie de l'AM, permettra de restaurer tout ou partie de la sensibilité des CCR à l'oxaliplatine. Les inventeurs ont en effet vérifié, par une étude de plusieurs mois de suivi des lignées cellulaires dérivées, que leur phénotype de résistance est spontanément réversible en l'absence de pression pharmacologique (= culture cellulaire sans oxaliplatine). Cette réversibilité, totale ou partielle selon les cas, permet de prévoir une réversion sous l'effet d'une substance contrariant ou contournant les mécanismes de résistance au sein de l'AM.

Les inventeurs ont en outre développé la purification de mitochondries à partir de lignées sensibles et résistantes afin d'isoler et de tester les effecteurs putatifs de la résistance (comme le PTPC), ainsi que des agents de blocage de ces effecteurs (ARN anti-sens, substances déjà connues pour bloquer un mécanisme physiologique au niveau de l'AM, ...). L'invention couvre également la mise en oeuvre de transferts de gène restaurant le phénotype de sensibilité à l'oxaliplatine, et de procédés de criblage de nouvelles entités chimiques permettant de contrarier ou contourner la résistance, à partir des effecteurs comme cibles et de banques de substances chimiques comme sources.

### EXEMPLE 2 : Activation de Bax et de Bak

### Activation de Bax

Bax est présent dans les cellules sous deux formes : une forme latente (inactive) et une forme active qui participe au processus apoptotique

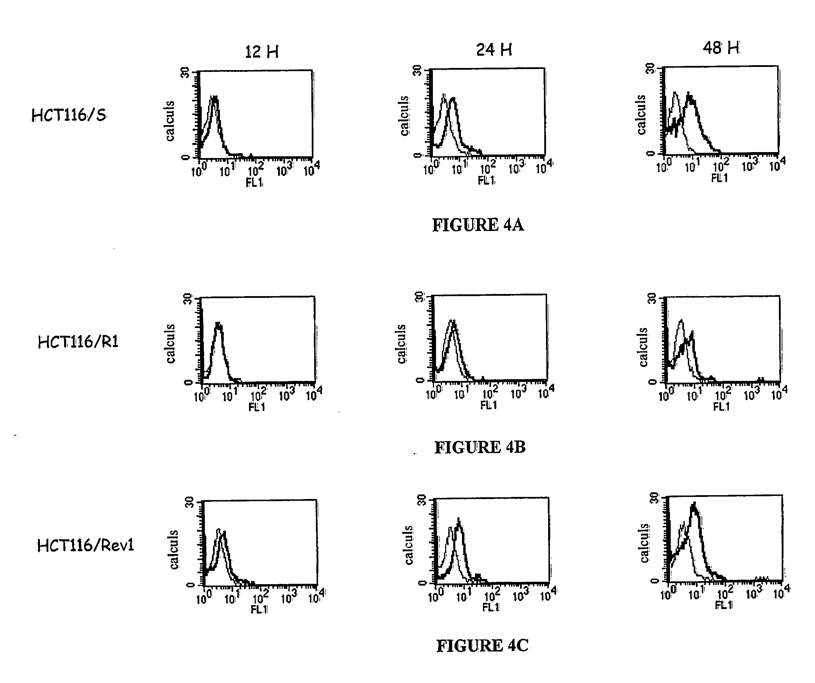

Le niveau global d'expression de Bax est équivalent pour les lignées HCT116/S, HCT116/R1 et HCT116/Rev1. L'exposition à l'oxaliplatine induit une surexpression de Bax. Cette surexpression demeurant comparable sur l'ensemble de ces lignées, les inventeurs ont cherché à savoir si le degré d'activation de Bax pouvait rendre compte de la réponse des cellules à l'oxaliplatine ou si définitivement Bax, pris isolément, ne pouvait pas être systématiquement associé à la sensibilité à l'oxaliplatine. Figures 4A à 4C: Sensibilité à l'oxaliplatine associée au degré d'activation de Bax

Légende des figures 4A à 4C : Détection de l'activation de Bax par un anticorps spécifique de la conformation active de Bax et analyse intracellulaire par cytométrie de flux. Les cellules sont fixées, perméabilisées puis incubées avec l'anticorps spécifique de la conformation active de Bax. Enfin la révélation se fait par l'incubation de l'anticorps secondaire couplé au FITC. Le marquage des cellules non traitées est représenté par la courbe en trait noir fin. Celui des cellules traitées (12, 24 ou 48 heures avec 15 uM d'oxaliplatine) est représenté par les courbes en trait noir épais. L'apparition de cellules fortement marquées (courbes en trait noir épais décalées sur la droite) témoigne de l'activation de Bax. L'expérience a été reproduite trois fois et a permis d'obtenir des résultats comparables.

Les inventeurs ont mis en évidence à l'aide des figures 4A à 4C que l'état de résistance ou sensibilité des cellules à l'oxaliplatine est finement corrélé avec le degré d'activation de Bax. L'activation est amoindrie et retardée dans la lignée résistante HCT116/R1 (figure 4B) par rapport à la lignée sensible HCT116/S (figure 4A). Le retour à l'état de sensibilité du révertant HCT116/Rev1 (figure 4C) s'accompagne du retour à une activation précoce de Bax. Activation de Bak

La molécule pro-apoptotique Bak joue un rôle prépondérant au même titre que Bax dans l'apoptose médiée par la perméabilisation mitochondriale. Ces deux molécules semblent avoir des fonctions très voisines et parfois redondantes. Les inventeurs ont donc également cherché à savoir s'il existait une relation entre niveau d'activation de Bak et une réponse à l'oxaliplatine sur l'ensemble des lignées y compris pour les lignées HCT116/R2 et Rev2 mutées sur Bax.

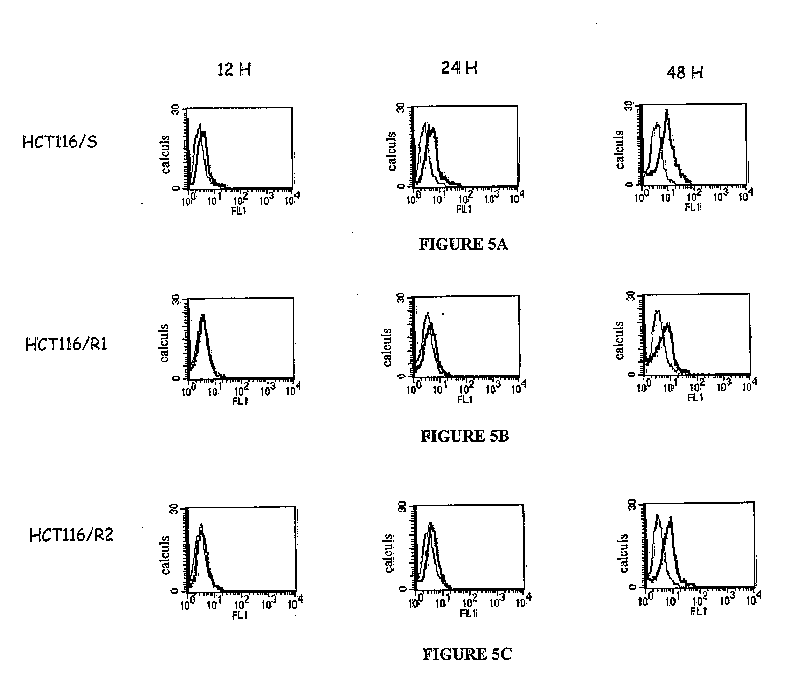

Figures 5A à 5E : Sensibilité à l'oxaliplatine associée au degré d'activation de Bak

Légendes des figures 5A à 5E : Détection de l'activation de Bak par un anticorps spécifique de la conformation active de Bak et analyse intracellulaire par cytométrie de flux. Les cellules sont fixées, perméabilisées puis incubées avec l'anticorps spécifique de la conformation active de Bax. Enfin la révélation se fait par l'incubation de l'anticorps secondaire couplé au FITC. Le marquage des cellules non traitées est représenté par la courbe en trait noir fin. Celui des cellules traitées (12, 24 ou 48 heures avec 15 uM d'oxaliplatine) est représenté par les courbes en trait noir épais. L'apparition de cellules fortement marquées (courbes en trait noir épais décalées sur la droite) témoigne de l'activation de Bak. Cette expérience a été reproduite deux fois.

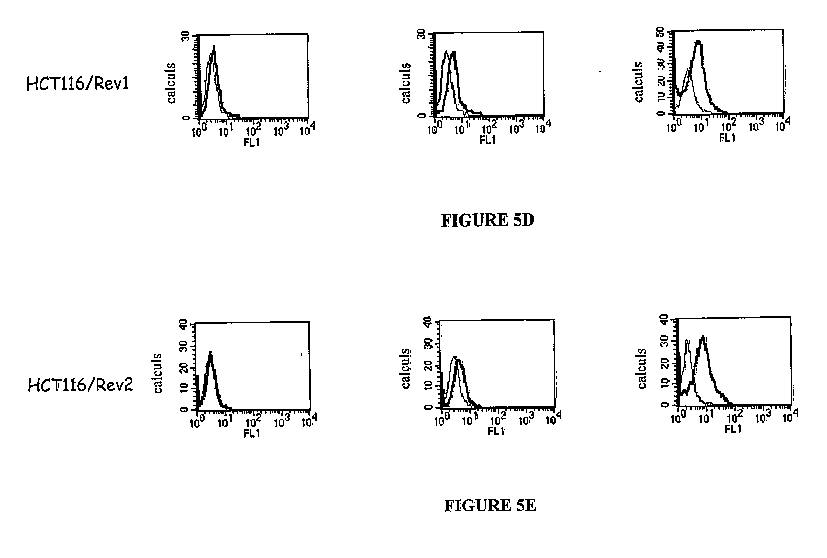

Les inventeurs ont mis en évidence à l'aide des figures 5A à 5E que L'activation de Bak en réponse au traitement par l'oxaliplatine est retardée sur les lignées résistantes HCT116/R1 (figure 5B) et HCT116/R2 (figure 5C). Les révertants Rev1 (figure 5D) et Rev2 (figure 5E) retrouvent le niveau d'activation de Bak de la lignée sensible HCT116/S (figure 5A). A l'égal de Bax, l'activation de Bak est finement corrélée à la réponse des cellules à l'oxaliplatine.

En conclusion, une co-activation de Bax et Bak peut être observée en réponse à l'oxaliplatine. L'activation précoce de Bax et Bak est associée à l'état de sensibilité des cellules à l'oxaliplatine.

## Revendications

1. Procédé de détection *in vitro* de la résistance de cellules cancéreuses à un traitement à l'oxaliplatine, **caractérisé en ce qu'**il comprend la mesure de l'altération de l'apoptose mitochondriale de cellules cancéreuses traitées ou pouvant ou devant être traitées à l'oxaliplatine, cette mesure comprenant au moins la mesure de l'expression du gène d'apoptose mitochondriale codant pour la protéine Bax.

2. Procédé selon la revendication 1, **caractérisé en ce que** le cancer est un cancer traité à l'oxaliplatine, notamment un cancer colorectal, un cancer des ovaires, un cancer des cellules germinales, un cancer du poumon, un cancer des voies digestives, un cancer de la prostate, un cancer du pancréas, un cancer de l'intestin grêle, un cancer de l'estomac.

3. Procédé selon la revendication 1 ou 2, **caractérisé en ce qu'**il comprend la mesure des ARNm transcrits du gène Bax d'apoptose mitochondriale.

4. Procédé selon la revendication 1 ou 2, **caractérisé en ce qu'**il comprend la mesure de la quantité et/ou de l'activité de la protéine Bax d'apoptose mitochondriale dans les cellules cancéreuses.

5. Procédé de détection *in vitro* de la résistance de cellules cancéreuses à un traitement à l'oxaliplatine **caractérisé en ce qu'**il comprend la détection d'au moins une mutation indicatrice d'une apoptose mitochondriale déficiente en cas de traitement à l'oxaliplatine dans une région du gène Bax contenant une série de 8 déoxyguanines.

6. Procédé selon l'une quelconque des revendications 1 à 4, **caractérisé en ce qu'**il comprend :
a) la détermination du niveau d'expression d'au moins du gène Bax d'apoptose mitochondriale, sur des cellules cancéreuses prélevées chez un patient ;
b) la comparaison du niveau mesuré avec un échantillon contrôle de cellules non résistantes à l'oxaliplatine.

7. Procédé selon la revendication 4, **caractérisé en ce qu'**il comprend la mise en contact des cellules cancéreuses avec un anticorps capable de reconnaître la protéine Bax d'apoptose mitochondriale ou un de ses fragments biologiquement actifs, et la mise en évidence du complexe antigène-anticorps éventuellement formé.

8. Procédé selon l'une quelconque des revendications 1 à 3, **caractérisé en ce qu'**il met en oeuvre une séquence amorce ou sonde spécifique du gène Bax d'apoptose mitochondriale.

9. Procédé selon la revendication 8, **caractérisé en ce qu'**il comprend :
a) éventuellement l'isolement de l'ADN mitochondrial à partir de l'échantillon biologique à examiner, ou l'obtention d'un ADNc à partir de l'ARN de l'échantillon biologique ou d'ADN génomique ;
b) l'amplification spécifique de l'ADN du a) à l'aide de l'amorce d'amplification spécifique du gène Bax d'apoptose mitochondriale.

10. Procédé selon la revendication 8, **caractérisé en ce qu'**il comprend :
a) la mise en contact d'une sonde nucléotidique du gène Bax d'apoptose mitochondriale avec l'échantillon biologique analysé, l'acide nucléique de l'échantillon ayant le cas échéant préalablement été rendu accessible à l'hybridation, dans des conditions permettant l'hybridation de la sonde et de l'acide nucléique de l'échantillon ;
b) la mise en évidence de l'hybride éventuellement formé.

11. Procédé de sélection *in vitro* de composés inhibiteurs de la résistance de cellules cancéreuses à l'oxaliplatine, **caractérisé en ce qu'**il comprend :
a) l'addition d'au moins un composé candidat à des cellules cancéreuses résistantes à l'oxaliplatine ;
b) la comparaison du niveau d'expression du gène Bax d'apoptose mitochondriale en présence et en absence du composé ;
c) la déduction de l'effet anti-résistance lorsque le niveau d'expression du gène Bax est supérieur après l'addition du composé.

## Claims

1. A method for *in vitro* detection of the resistance of cancer cells to a treatment with oxaliplatin, **characterized in that** it comprises the measurement of the alteration of mitochondrial apoptosis of cancer cells treated or being able or having to be treated with oxaliplatin, this measurement comprising at least the measurement of the expression of the mitochondrial apoptosis gene coding for the Bax protein.

2. The method according to claim 1,
**characterized in that** the cancer is a cancer treated with oxaliplatin, notably colorectal cancer, ovary cancer, germinal cells cancer, lung cancer, digestive tract cancer, prostate cancer, pancreas cancer, small intestine cancer, stomach cancer.

3. The method according to claim 1 or 2,
**characterized in that** it comprises the measurement of mRNA transcribed from the mitochondrial apoptosis Bax gene.

4. The method according to claim 1 or 2, **characterized in that** it comprises the measurement of the amount and/or activity of the mitochondrial apoptosis Bax protein in cancer cells.

5. A method for *in vitro* detection of the resistance of cancer cells to a treatment with oxaliplatin, **characterized in that** it comprises the detection of at least one mutation indicating deficient mitochondrial apoptosis in the case of a treatment with oxaliplatin in a region of the Bax gene containing a series of 8 deoxyguanines.

6. The method according to any of claims 1 to 4, **characterized in that** it comprises:
a) determining the expression level of at least the mitochondrial apoptosis Bax gene, on cancer cells sampled from a patient;
b) comparing the measured level with a control sample of cells non-resistant to oxaliplatin.

7. The method according to claim 4,
**characterized in that** it comprises the contacting of cancer cells with an antibody capable of recognizing the mitochondrial apoptosis Bax protein or one of its biologically active fragments, and the detection of the possibly formed antigen-antibody complex.

8. The method according to any of claims 1 to 3, **characterized in that** it applies a primer sequence or probe specific to the mitochondrial apoptosis Bax gene.

9. The method according to claim 8,
**characterized in that** it comprises:
a) optionally isolating mitochondrial DNA from the biological sample to be examined, or obtaining cDNA from the RNA of the biological sample or from genomic DNA;
b) specifically amplifying the DNA from a) by means of the primer for specifically amplifying the mitochondrial apoptosis Bax gene.

10. The method according to claim 8,
**characterized in that** it comprises:
a) contacting a nucleotide probe from the mitochondrial apoptosis Bax gene with the analyzed biological sample, the nucleic acid of the sample having been, if necessary, made accessible beforehand to hybridization, under conditions allowing hybridization of the probe and of the nucleic acid of the sample;
b) detecting the possibly formed hybrid.

11. A method for *in vitro* selection of compounds inhibiting the resistance of cancer cells to oxaliplatin, **characterized in that** it comprises:
a) adding at least one candidate compound to cancer cells resistant to oxaliplatin;
b) comparing the expression level of the mitochondrial apoptosis Bax gene in the presence and in the absence of the compound;
c) inferring the anti-resistance effect when the expression level of the Bax gene is higher after adding the compound.

## Patentansprüche

1. In vitro-Nachweisverfahren der Resistenz von Krebszellen auf eine Behandlung mit Oxaliplatin, **dadurch gekennzeichnet, dass** es das Messen der Veränderung der mitrochondrialen Apoptose von Krebszellen umfasst, die mit Oxaliplatin behandelt wurden oder werden können oder müssen, wobei diese Messung mindestens das Messen der Expression des Gens der mitrochondrialen Apoptose umfasst, das für das Bax-Protein codiert.

2. Verfahren nach Anspruch 1, **dadurch gekennzeichnet, dass** das Karzinom ein mit Oxaliplatin behandeltes Karzinom ist, vor allem ein kolorektales Karzinom, ein Karzinom der Eierstöcke, ein Karzinom der Keimzellen, ein Karzinom der Lunge, ein Karzinom der Verdauungskanäle, ein Karzinom der Prostata, ein Karzinom der Bauspeicheldrüse, ein Karzinom des Dünndarms, ein Karzinom des Magens.

3. Verfahren nach Anspruch 1 oder 2, **dadurch gekennzeichnet, dass** es das Messen der transkribierten mRNAs des Bax-Gens der mitrochondrialen Apoptose umfasst.

4. Verfahren nach Anspruch 1 oder 2, **dadurch gekennzeichnet, dass** es das Messen der Menge und/oder der Aktivität des Bax-Proteins der mitrochondrialen Apoptose in den Krebszellen umfasst.

5. In vitro-Nachweisverfahren der Resistenz von Krebszellen auf eine Behandlung mit Oxaliplatin, **dadurch gekennzeichnet, dass** es den Nachweis von mindestens einer Mutation umfasst, die auf eine defiziente mitrochondriale Apoptose bei einer Behandlung mit Oxaliplatin in einer Region des Bax-Gens mit einer Reihe von 8 Deoxyguaninen umfasst.

6. Verfahren nach einem der Ansprüche 1 bis 4, **dadurch gekennzeichnet, dass** es umfasst:
a) die Bestimmung des Niveaus der Expression mindestens eines Bax-Gens der mitrochondrialen Apoptose auf den einem Patienten entnommenen Krebszellen,
b) den Vergleich des gemessenen Niveaus mit einer Kontrollprobe von Zellen, die nicht oxaliplantinresistent sind.

7. Verfahren nach Anspruch 4, **dadurch gekennzeichnet, dass** es die Herstellung des Kontakts der Krebszellen mit einem Antikörper umfasst, der imstande ist, das Bax-Gen der mitrochondrialen Apoptose oder eines seiner biologisch aktiven Fragmente zu erkennen, und den Nachweis des eventuell gebildeten Antigen-Antikörper-Komplexes.

8. Verfahren nach einem der Ansprüche 1 bis 3, **dadurch gekennzeichnet, dass** es eine Primersequenz oder spezifische Sonde des Bax-Gens der mitrochondrialen Apoptose umsetzt.

9. Verfahren nach Anspruch 8, **dadurch gekennzeichnet, dass** es umfasst:
a) eventuell die Isolierung der mitrochondrialen DNA aus der zu prüfenden biologischen Probe, oder die Herstellung einer cDNA aus der RNA der biologischen Probe oder der Genom-DNA,
b) die spezifische Amplifizierung der DNA von a) mit Hilfe des spezifischen Amplifikationsprimers des Bax-Gens der mitrochondrialen Apoptose.

10. Verfahren nach Anspruch 8, **dadurch gekennzeichnet, dass** es umfasst:
a) die Herstellung des Kontakts einer Nukleotidsonde des Bax-Gens der mitrochondrialen Apoptose mit der analysierten biologischen Probe, wobei die Nukleinsäure der Probe gegebenenfalls vorher für die Hybridisierung zugänglich gemacht wurde, unter Bedingungen, die die Hybridisierung der Sonde und der Nukleinsäure der Probe erlauben,
b) den Nachweis des eventuell gebildeten Hybrids.

11. In vitro-Selektionsverfahren von Verbindungen, die die Oxaliplatinresistenz von Krebszellen inhibieren, **dadurch gekennzeichnet, dass** es umfasst:
a) die Zugabe mindestens einer Kandidatverbindung zu den oxaliplatinresistenten Krebszellen,
b) den Vergleich des Expressionsniveaus des Bax-Gens der mitrochondrialen Apoptose bei Anwesenheit und in Abwesenheit der Verbindung,
c) den Rückschluss auf die Anti-Resistenz-Wirkung, wenn das Expressionsniveau des Bax-Gens nach Zugabe der Verbindung höher ist.
